# EUROPEAN PATENT APPLICATION

(11) **EP 4 406 513 A1**
(43) Date of publication of application: **31.07.2024**
(21) Application number: 24151882.8
(22) Date of filing: 15.01.2024
(51) Int. Cl.: A61F 2/24

(54) **REINFORCED COMMISSURE FOR PROSTHETIC HEART VALVE**

(30) Priority: 27.01.2023 US 202363481895 P; 30.11.2023 US 202318524192
(71) Applicant: Medtronic, Inc., Minneapolis, MN 55432 (US)
(72) Inventor: OLNEY, Karl, Santa Ana, 92705 (US)
(74) Representative: Zimmermann & Partner Patentanwälte mbB

(57) **Abstract**

A transcatheter heart valve prosthesis a frame and a prosthetic valve having a plurality of leaflets. Each leaflet includes a first and second lateral end. The transcatheter heart valve prosthesis further includes a commissure backing. The commissure backing includes a first portion disposed between a lateral end of a first leaflet and a lateral end of a second leaflet. The first portion of the commissure backing and the lateral ends of the first and second leaflets are joined together. Each commissure backing further includes a second portion that extends radially outwardly from the first portion of the commissure backing and is coupled to the frame.

## Description

### CROSS-REFERENCE TO RELATED APPLICATIONS

This application claims the benefit of U.S. Provisional Application Serial No. 63/481,895, filed January 27, 2023.

### FIELD OF THE INVENTION

The present disclosure relates to medical devices. More particularly, the present disclosure is related to a transcatheter heart valve prosthesis and a method of assembling the transcatheter heart valve prosthesis.

### BACKGROUND

The human heart is a four chambered, muscular organ that provides blood circulation through the body during a cardiac cycle. The four main chambers include the right atrium and right ventricle which supplies the pulmonary circulation, and the left atrium and left ventricle which supplies oxygenated blood received from the lungs into systemic circulation. To ensure that blood flows in one direction through the heart, atrioventricular valves (tricuspid and mitral valves) are present between the junctions of the atrium and the ventricles, and semi-lunar valves (pulmonary valve and aortic valve) govern the exits of the ventricles leading to the lungs and the rest of the body. These valves contain leaflets or cusps that open and shut in response to blood pressure changes caused by the contraction and relaxation of the heart chambers. The valve leaflets move apart from each other to open and allow blood to flow downstream of the valve, and coapt to close and prevent backflow or regurgitation in an upstream manner.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

Commissures of a heart valve prosthesis refer to the locations where the adjacent leaflets meet and attach to each other. Generally, the commissures of the leaflets are also attached to the frame of the heart valve prosthesis. The commissure section of a heart valve is typically where the highest stresses are located for valve loading.

The present disclosure relates to improvements of commissure construction that utilizes a fabric backing configured to strengthen the commissure by increasing the suture pull out strength, reduce the bulk of the commissure which will reduce the heart valve prosthesis profile and increase the opening area of the valve, which in turn, improves the effective orifice area (EOA).

### BRIEF SUMMARY OF THE INVENTION

In accordance with first example hereof, a transcatheter heart valve prosthesis includes a frame, a prosthetic valve having a plurality of leaflets each including lateral ends, and a commissure backing. The commissure backing includes a first portion and a second portion. The first disposed between a first lateral end of a first leaflet of the plurality of leaflets and a second lateral end of a second leaflet of the plurality of leaflets, wherein the first lateral end, the second lateral end, and the first portion of the commissure backing are joined together. The second portion extends radially outwardly from the first and second lateral ends and is coupled to the frame.

In a second example, the transcatheter heart valve prosthesis of any of the previous or subsequent examples further includes a reinforcing member, wherein the reinforcing member is joined to the first lateral end, second lateral end, and the first portion of the commissure backing.

In a third example, in the transcatheter heart valve prosthesis of any of the previous or subsequent examples, the reinforcing member comprises tissue, polytetrafluoroethylene (PTFE), hydrogels, and/or polymers.

In a fourth example, in the transcatheter heart valve prosthesis of any of the previous or subsequent examples, the reinforcing member includes a fold.

In a fifth example, in the transcatheter heart valve prosthesis of any of the previous or subsequent examples, the reinforcing member includes a first portion disposed on an outer surface of the first lateral end of the first leaflet and a second portion disposed on an outer surface of the second lateral end of the second leaflet, wherein the fold is disposed between the first and second portions of the leaflet, and wherein the reinforcing member is joined to the first lateral end, the second lateral end, and the first portion of the commissure backing via sutures extending from the first portion of the reinforcing member to the second portion of the reinforcing member through the first lateral end, the second lateral end, and the first portion of the commissure backing.

In a sixth example, in the transcatheter heart valve prosthesis of any of the previous or subsequent examples, the commissure backing is folded such that the first portion of the commissure backing comprises two layers disposed between first lateral end of the first leaflet and the second lateral end of the second leaflet.

In a seventh example, in the transcatheter heart valve prosthesis of any of the previous or subsequent examples, the commissure backing includes at least one cutout configured to allow a strut of the frame to extend therethrough.

In an eighth example, in the transcatheter heart valve prosthesis of any of the previous or subsequent examples, the frame includes a slot commissure post, wherein the slot commissure post includes a first axial strut, a second axial strut, and an opening disposed between the first and second axial struts.

In a ninth example, in the transcatheter heart valve prosthesis of any of the previous or subsequent examples, the commissure backing is folded such that the first portion of the commissure backing comprises two layers disposed between first lateral end of the first leaflet and the second lateral end of the second leaflet, and the second portion of the commissure backing includes a first end extending from the fold and a second end extending from the fold.

In a tenth example, in the transcatheter heart valve prosthesis of any of the previous or subsequent examples, the second portion of the commissure backing extends radially outwardly through the opening of the slot commissure post such that the first portion of the commissure backing, the first lateral end of the first leaflet, and the second lateral end of the second leaflet are disposed within an interior of the frame.

In an eleventh example, in the transcatheter heart valve prosthesis of any of the previous or subsequent examples, the second portion of the commissure backing is wrapped around the first axial strut and second axial strut of the slot commissure post.

In a twelfth example, in the transcatheter heart valve prosthesis of any of the previous or subsequent examples, the commissure backing comprises polyethylene terephthalate (PET), polytetrafluoroethylene (PTFE), and/or polyester.

In a thirteenth example, a method of assembling a transcatheter heart valve prosthesis includes: positioning a first portion of a commissure backing between a first lateral end of a first leaflet and a second lateral end of a second leaflet, the second leaflet being adjacent to the first leaflet, wherein a second portion of the commissure backing extends radially outwardly from the first portion; attaching the first portion of the commissure backing to the first lateral end of the first leaflet and the second lateral end of the second leaflet; and attaching the second portion of the commissure backing to a commissure post of a frame.

In a fourteenth example, the method of any of the previous or subsequent examples further includes attaching a reinforcing member to the first lateral end of the first leaflet, the second lateral end of the second leaflet, and the first portion of the commissure backing.

In a fifteenth example, in the method of any of the previous or subsequent examples, attaching the reinforcing member comprises positioning a first portion of the reinforcing member on an outer surface of the first lateral end of the first leaflet, positioning a second portion of the reinforcing member on an outer surface of the second lateral end of the second leaflet, with a fold disposed between the first and second portions of the reinforcing member, and joining the reinforcing member to the first lateral end, the second lateral end, and the first portion of the commissure backing via sutures extending from the first portion of the reinforcing member to the second portion of the reinforcing member through the first lateral end, the second lateral end, and the first portion of the commissure backing.

In a sixteenth example, the method of any of the previous or subsequent examples further includes folding the commissure backing to form the first portion of the commissure backing and the second portion of the commissure backing.

In a seventeenth example, the method of any of the previous or subsequent examples further includes aligning a cutout of the commissure backing with a strut of the frame such that the strut extends through the cutout.

In an eighteenth example, in the method of any of the previous or subsequent examples, attaching the second portion of the commissure backing to the commissure post comprises attaching the second portion of the commissure backing to a slot commissure post that includes a first axial strut, a second axial strut, and an opening disposed between the first and second axial struts.

In a nineteenth example, in the method of any of the previous or subsequent examples, attaching the second portion of the commissure backing to the slot commissure post comprises extending the second portion of the commissure backing extends radially outwardly through the opening of the slot commissure post such that the first portion of the commissure backing, the first lateral end of the first leaflet, and the second lateral end of the second leaflet are disposed within an interior of the frame.

In a twentieth example, the method of any of the previous or subsequent examples further includes wrapping the second portion of the commissure backing at least partially around the first axial strut and second axial strut of the slot commissure post, and attached the second portion of the commissure backing to the slot commissure post.

Further disclosed herein is a transcatheter heart valve prosthesis a frame and a prosthetic valve having a plurality of leaflets, wherein each leaflet includes a first and second lateral end, wherein the transcatheter heart valve prosthesis further includes a commissure backing, wherein the commissure backing includes a first portion disposed between a lateral end of a first leaflet and a lateral end of a second leaflet, wherein the first portion of the commissure backing and the lateral ends of the first and second leaflets are joined together, wherein each commissure backing further includes a second portion that extends radially outwardly from the first portion of the commissure backing and is coupled to the frame.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 illustrates an exemplary side view of a transcatheter heart valve prosthesis according to embodiments hereof.
FIG. 2 illustrates an exemplary leaflet of a transcatheter heart valve prosthesis according to embodiments hereof.
FIG. 3A illustrates a commissure backing of a transcatheter heart valve prosthesis according to embodiments hereof.
FIG. 3B illustrates the commissure backing of FIG. 3A in a folded configuration according to embodiments hereof.
FIG. 4A illustrates a reinforcing member of a transcatheter heart valve prosthesis according to embodiments hereof.
FIG. 4B illustrates the reinforcing member of FIG. 4A in a folded configuration according to embodiments hereof.
FIG. 5 is a block diagram that shows a method of coupling a prosthetic heart valve to a frame of a heart valve prosthesis according to embodiments hereof.
FIG. 5A illustrates a step in the method of FIG. 5, showing a first portion of the commissure backing being aligned between a first lateral end of a first leaflet and a second lateral end of a second leaflet according to embodiments hereof.
FIG. 5B illustrates a step in the method of FIG. 5, showing a reinforcing member being placed over the first lateral end of the first leaflet, the second lateral end of the second leaflet, and the first portion of the commissure backing according to embodiments hereof.
FIG. 5C illustrates a step in the method of FIG. 5, showing the reinforcing member being attached to the first lateral end of the first leaflet, the second lateral end of the second leaflet, and the first portion of the commissure backing according to embodiments hereof.
FIGS. 5D-5E illustrate a step in the method of FIG. 5, showing a second portion of the commissure backing being attached to a commissure post of a frame of a heart valve prosthesis according to embodiments hereof.
FIGS. 6A-6F illustrate various ways of attaching the second portion of the commissure backing to a commissure post of a frame of a heart valve prosthesis according to embodiments hereof.
FIGS. 7A-7B illustrate various ways of attaching the second portion of the commissure backing to a commissure post of a frame of a heart valve prosthesis according to embodiments hereof.
FIG. 8 illustrates an exemplary slot commissure post of a frame of a heart valve prosthesis according to embodiments hereof.
FIGS. 8A-8D illustrate various ways of attaching the second portion of the commissure backing to the slot commissure post of FIG. 8 according to embodiments hereof.
FIG. 9A illustrates an alternate commissure backing in an as-cut state, according to embodiments hereof.
FIG. 9B illustrates the commissure backing of FIG. 9A attached to an open cell of a frame of a heart valve prosthesis according to embodiments hereof.
FIG. 10A illustrates an alternate embodiment of a transcatheter heart valve prosthesis, wherein the second portion of the commissure backing is coupled to a skirt of the transcatheter heart valve prosthesis according to embodiments hereof.
FIG. 10B illustrates a close-up view of the commissure backing coupled to the skirt of the transcatheter heart valve prosthesis of FIG. 10A according to embodiments hereof.
FIG. 10C illustrates a side view of the transcatheter heart valve prosthesis of FIG. 10A according to embodiments hereof.
FIG. 10D illustrates a close up view of the transcatheter heart valve prosthesis of FIG. 10C according to embodiments hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery device. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a," "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

Further, numerical terms such as "first," "second," "third," etc. used herein are not meant to be limiting such that use of the term "second" when referring to a part in the specification does not mean that there necessarily is a "first" of part in order to fall within the scope of the invention. Instead, such numbers are merely describing that the particular embodiment being described has a "first" part and a "second" part. The invention is instead defined by the claims, in which one or more of the numbered parts may be claimed.

Specific embodiments of the present invention are now described with reference to the figures. The terms "inflow" and "outflow", when used in the following description refer to a native vessel, native valve, or a device to be implanted into a native vessel or native valve, such as a heart valve prosthesis, are with reference to the direction of blood flow. Thus, "inflow" refers to positions in an upstream direction with respect to the direction of blood flow and the term "outflow" refers to positions in a downstream direction with respect to the direction of blood flow. Similarly, the terms "distal" and "distally" when used with respect to a heart valve prosthesis refer to positions in a downstream direction, and the terms "proximal" and "proximally" refer to positions in an upstream direction.

The heart valve prosthesis described herein is configured for treating a native aortic valve. However, the devices and methods described herein can be used in conjunction with transcatheter heart valve prostheses for treating other heart valves, such as those configured for treating native mitral valves, tricuspid valves, and pulmonary valves.

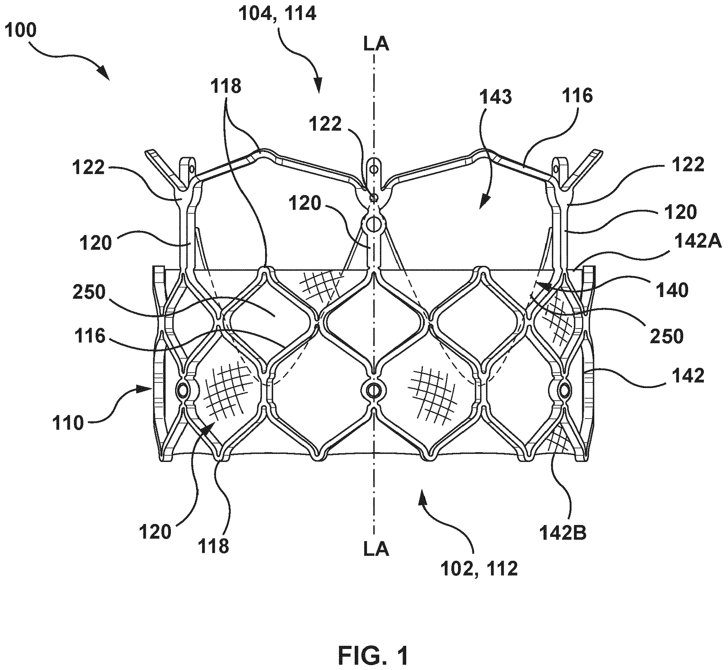

FIG. 1 shows a side view of an example transcatheter heart valve prosthesis 100. Those skilled in the art would recognize that the transcatheter heart valve prosthesis 100 is exemplary in nature and that other transcatheter heart valve prostheses may be used in keeping with the principles of the present invention. In the example shown, the transcatheter heart valve prosthesis 100 includes an inflow end 102 and an outflow end 104. The transcatheter heart valve prosthesis 100 generally includes a frame 110 and a prosthetic valve 140. The frame 110 of the transcatheter heart valve prosthesis 100 is an expandable stent-like frame comprised of a plurality of thin interconnecting members referred to herein as struts 116, arranged in a variety of geometrical patterns. The frame 110 further includes an inflow end 112 that defines the inflow end 102 of the transcatheter heart valve prosthesis 100 and an outflow end 114 that defines the outflow end 104 of the transcatheter heart valve prosthesis 100 and opposes the inflow end 102.

The frame 110 of the transcatheter heart valve prosthesis 100 generally forms a hollow cylindrical shape around a central longitudinal axis L_{A}, having a substantially constant diameter from the inflow end 112 to the outflow end 114 thereof, and defining a central lumen 143 between the inflow end 112 and the outflow end 114. However, this is not meant to be limiting, and frames with various diameters may also be used. The struts 116 of the frame 110 define a plurality of open cells 120 arranged in a fence-like or honeycomb pattern. Further, the frame 110 includes a plurality of crowns 118 where the struts 116 meet. The frame 110 further includes commissure posts 122 for attachment of the plurality of leaflets, as described in further detail below. The commissure posts 122 are struts 116 of the frame 110 that are arranged vertically, i.e., parallel to the central longitudinal axis L_{A}. In other embodiments, there may be additional longitudinal struts (not shown) disposed between adjacent commissure posts 122.

In the embodiment shown, the transcatheter heart valve prosthesis 100 further includes a skirt 142 coupled to the frame 110. More particularly, in the embodiment shown, the skirt lines a portion of an interior surface of the frame 110. However, this is not meant to be limiting, as the skirt 142 may alternatively be coupled to an exterior surface of the frame 110. The skirt 142 includes a first end 142A and a second end 142B opposite the first end 142A. When the transcatheter heart valve prosthesis 100 is implanted within a native valve, the skirt 142 is configured to limit the amount of unintentional blood leakage, otherwise known as regurgitation or paravalvular leakage. In particular, the skirt 142 is configured to limit blood flow around the transcatheter heart valve prosthesis 100 such that blood flows from the inflow end 102 to the outflow end 104 only when the transcatheter heart valve prosthesis 100 is in an open state. The skirt 142 may take the form of a single piece or multiple pieces of material that is/are wrapped within the interior surface as to create a cylindrical body that is flush with the interior surface. The skirt 142 may be affixed to the frame 110 by using sutures or adhesives. In order to inhibit blood flow, the skirt 142 is further configured to substantially cover the cells 120 of the frame 110 disposed between the first end 142A and the second end 142B of the skirt 142.

The prosthetic valve 140 includes a plurality of leaflets 250. In the embodiment shown, the prosthetic valve 140 includes three leaflets 250, but this is not meant to be limiting. The prosthetic valve 140 is capable of regulating flow through the frame 110 via the valve leaflets 250. When deployed *in situ,* the transcatheter heart valve prosthesis 100 in a closed state is configured to block blood flow in one direction to regulate blood flow through the lumen 143 of the frame 110. The valve leaflets 250 are disposed within the frame 110 such that free edges of the leaflets 250 coapt to close the prosthetic valve 140, and separate to open the prosthetic valve 140. Adjoining pairs of leaflets are attached to one another at their lateral ends to form leaflet commissures, as described in more detail below. The commissures are coupled to corresponding commissure posts 120, as described in more detail below. Bases of the leaflets 250 may be coupled to the skirt 142 and/or the frame 110. The orientation of the leaflets 250 within the frame 110 depends upon which end of the transcatheter heart valve prosthesis 100 is the inflow end 102 and which end of the transcatheter heart valve prosthesis 100 is the outflow end 104, thereby ensuring one-way flow of blood through the transcatheter heart valve prosthesis 100.

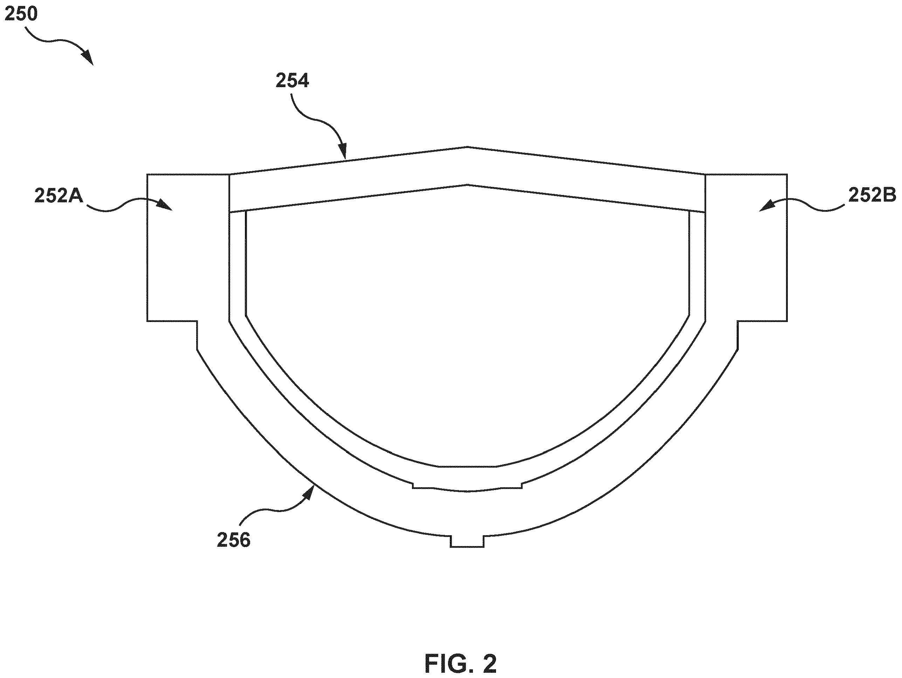

FIG. 2 shows an example leaflet 250 of the prosthetic valve 140 in an as-cut configuration according to embodiments hereof. In the embodiment shown, each leaflet 250 includes a first lateral tab or end 252A and a second lateral tab or end 252B opposite the first lateral end 252A. The first and second lateral ends 252A, 252B may be substantially rectangular in shape, but that is not meant to be limiting. Each leaflet 250 further includes a free edge 254 that extends from the first lateral end 252A to the second lateral end 252B and a base edge 256 that extends from the first lateral end 252A to the second lateral end 252B disposed opposite the free edge 254 of the leaflet 250. The fee edge 254 and the base edge 256 define the "top" and "bottom" edges of the leaflet 250. The base edges 256 of the leaflets 250 may be coupled to the frame 110 and/or skirt 142 of the transcatheter heart valve prosthesis 100. The free edges 254 of the leaflets 250 are unattached to the frame 110 or skirt 142 and are disposed within the lumen 143 of the frame 110. When the transcatheter heart valve prosthesis 100 is deployed, the free edges 254 of the leaflets 250 meet or coapt to close the prosthetic valve 140, and separate from each other to open the prosthetic valve 140.

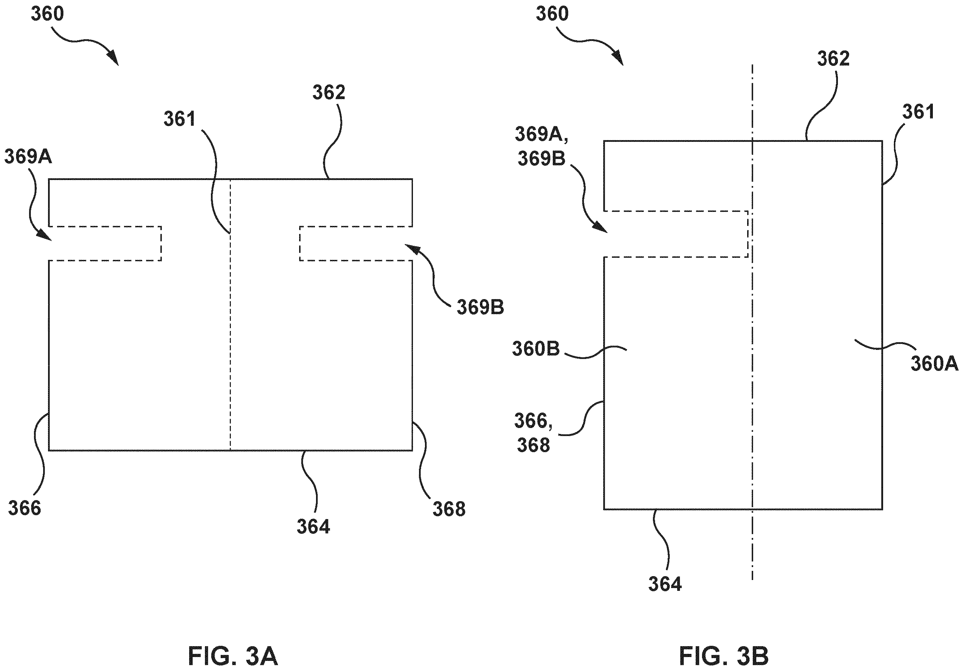

The prosthetic valve 140 of the transcatheter heart valve prosthesis 100 further includes one or more commissure backings 360, as shown in FIG. 3A. In particular, each commissure formed at adjoining leaflets 250 may include a commissure backing 360. Each commissure backing 360 may be a substantially rectangular-shaped piece of fabric that includes an upper edge 362, a lower edge 364 that runs parallel to and opposite the upper edge 362, a first side edge 366 that extends perpendicularly from the upper edge 362 to the lower edge 364, and a second side edge 368 that extends from the upper edge 362 to the lower edge 364 and runs parallel to and opposite the first side edge 366. Each commissure backing 360 may also include one or more cutouts 369A, 369B that are configured to allow one or more struts 116 of the frame 110 to extend therethrough, which is explained in further detail below. However, in other embodiments, the cutouts are not necessary. In the embodiment shown, a first cutout 369A and a second cutout 369B may be rectangular in shape and formed from the first and second side edges 366, 368, as shown in FIG. 3. The first and second cutouts 369A, 369B may be aligned such that they overlap when the commissure backing 360 is folded in half, which is described in further detail below. The particular shape of the cutouts is not meant to be limiting, and the cutouts may be different sizes, shapes, and locations depending on several factors, such as the location of the struts on the frame.

As previously stated, the commissure backing 360 is folded in half along a fold line 361, shown in FIG. 3A, that runs through a center of the upper and lower edges 362, 364 of the commissure backing 360. FIG. 3B shows the commissure backing 360 in a folded configuration, i.e., when it is folded along the fold line 361. As can be seen, the first side edge 366 and the second side edge 368 of the commissure backing 360 align or overlap in the folded configuration. Similarly, the first cutout 369A and the second cutout 369B of the commissure backing 360 align or overlap in the folded configuration. A longitudinal portion of the commissure backing 360 that includes the fold line 361 and does not include the first and second cutouts 369A, 369B is referred to herein as a first portion 360A of the commissure backing 360. A longitudinal portion of the commissure backing 360 that includes the first and second side edges 366, 368 and contains or overlaps with the first and second cutouts 369A, 369B is referred to herein as a second portion 360B of the commissure backing, as shown in FIG. 3B when the commissure backing 360 is in the folded configuration. In other embodiments, the first and second portions 360A, 360B can be defined by a first portion disposed between the lateral ends 252A, 252B of the adjacent leaflets 250 and a second portion for coupling to the frame 110 of the heart valve prosthesis 100, as described in further detail below. Further, in other embodiments, the commissure backing 360 need not be folded such that the first portion 360A is defined as being disposed between the lateral ends 252A, 252B of the adjacent leaflets 250 and the second portion for coupling to the frame 110 of the heart valve prosthesis 100. The first and second portions 360A, 360B of the commissure backing 360 may be substantially rectangular in shape. The first portion 360A of the commissure backing 360 is configured to attach to the lateral ends 252A, 252B of adjacent leaflets 250, while the second portion 360B of the commissure backing 360 is configured to attach to the commissure posts 122 of the frame 110, which will be described in further detail below. The commissure backing 360 may comprise woven or knitted fabrics, such as but not limited to, polyethylene terephthalate (PET), polytetrafluoroethylene (PTFE), and/or polyester. The commissure backing 360 may instead comprise tissue, such as, but not limited to, pericardial tissue.

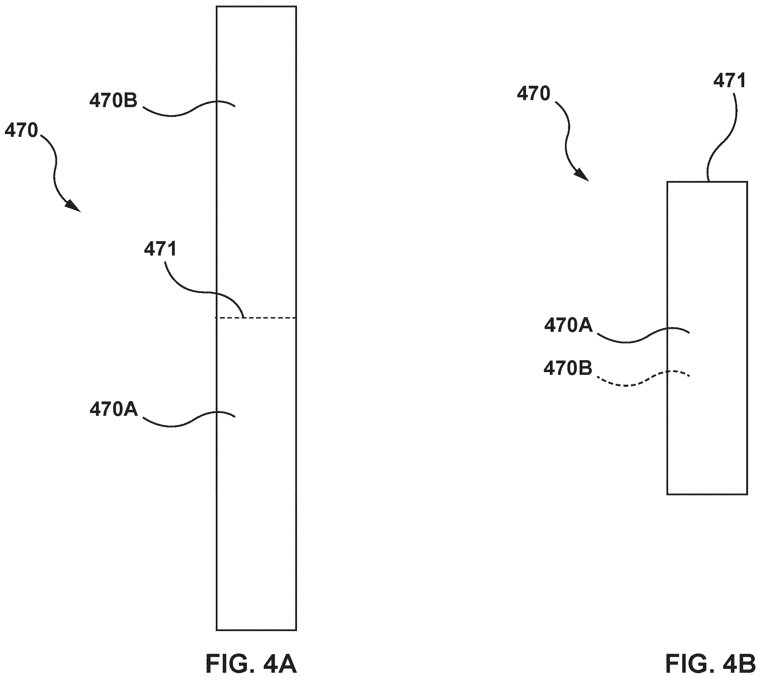

The prosthetic valve 140 of the transcatheter heart valve prosthesis 100 may further include one or more reinforcing members 470, as shown in FIG. 4A. Each reinforcing member 470 may be associated with corresponding commissure of the prosthetic valve 140. Accordingly, in an embodiment with three leaflets 250, and hence three commissures, the prosthetic valve 140 may include three reinforcing members 470. In an embodiment, each reinforcing member 470 is substantially rectangular including an upper edge, a lower edge that runs parallel to and opposite the upper edge, a first side edge that extends from the upper edge to the lower edge, and a second side edge that extends from the upper edge to the lower edge and runs parallel to and opposes the first side edge. The reinforcing member 470 may be folded in half along a fold line 471, shown in FIG. 4A, that runs through a center of the first and second side edges of the reinforcing member 470. The fold line 471 folds the reinforcing member 470 into two equal halves, referred to herein as a first portion 470A and a second portion 470B of the reinforcing member 470. FIG. 4B shows the reinforcing member 470 in a folded configuration, *i.e.,* when the reinforcing member 470 is folded along the fold line 471. As can be seen, the upper edge and the lower edge of the reinforcing member 470 align or overlap in the folded configuration. Similarly, the first portion 470A and the second portion 470B of the reinforcing member 470 align or overlap in the folded configuration. It is noted that the terms "upper", "lower", and "side" are used herein in the orientation shown, and are not meant to be limiting. The reinforcing member 470 preferably may comprise tissue, such as, but not limited to, pericardial tissue. However, this is not meant to be limiting, and other materials, such as, but not limited to, polytetrafluoroethylene (PTFE), hydrogels, and polymers with or without functionalized coatings such as Hyaluronic acid.

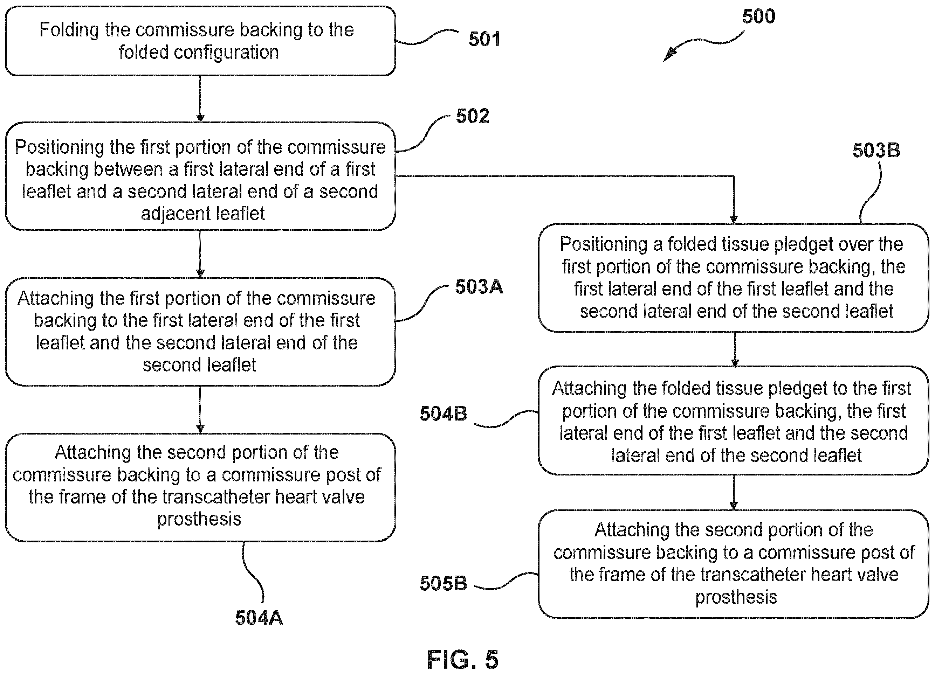

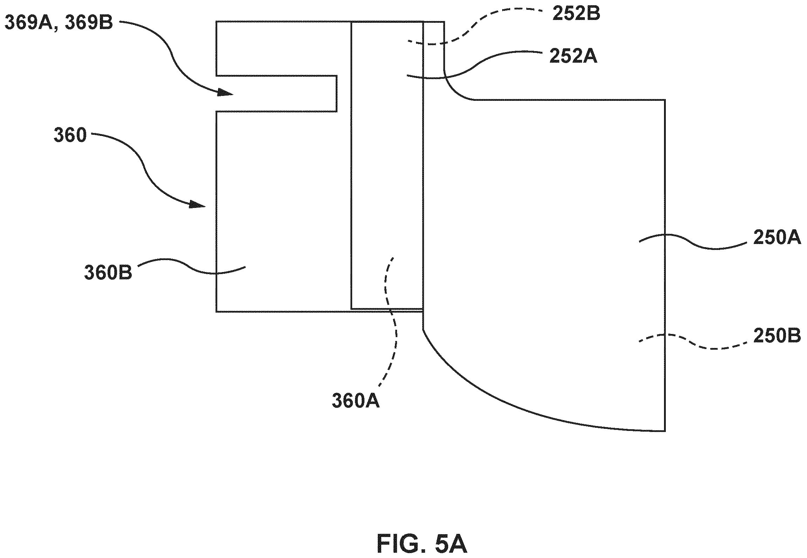

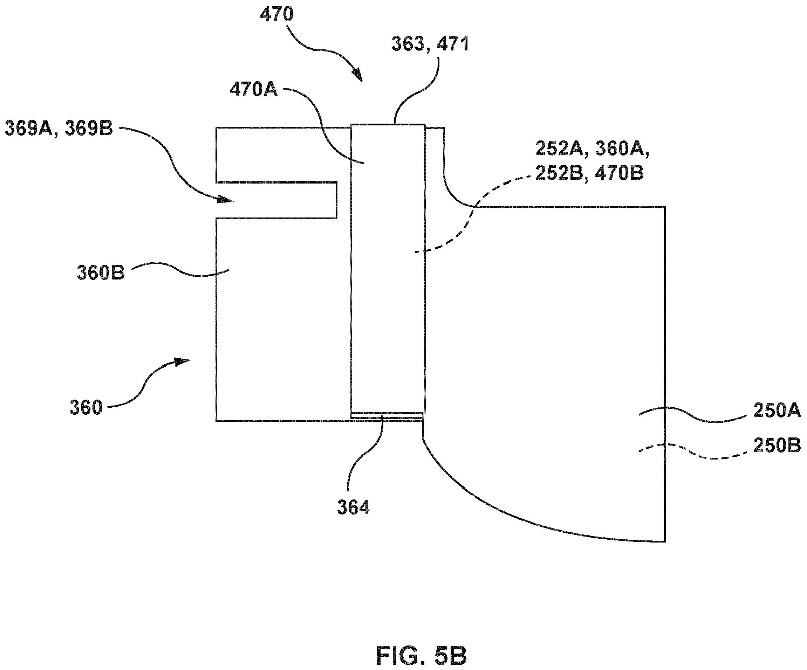

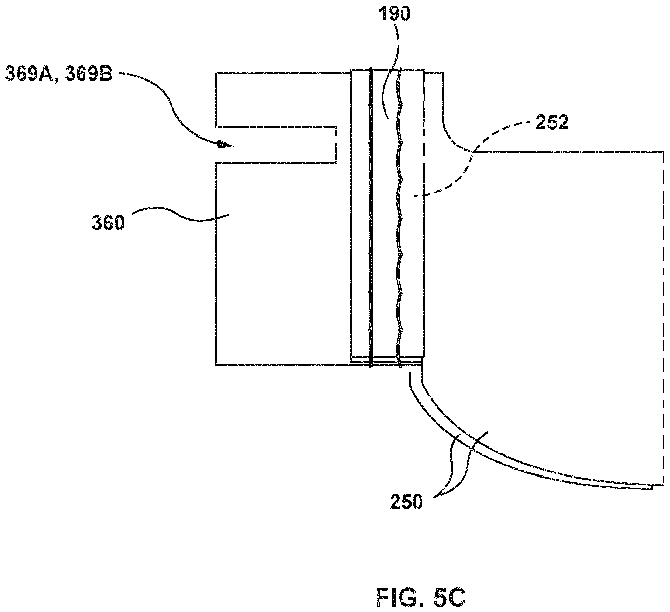

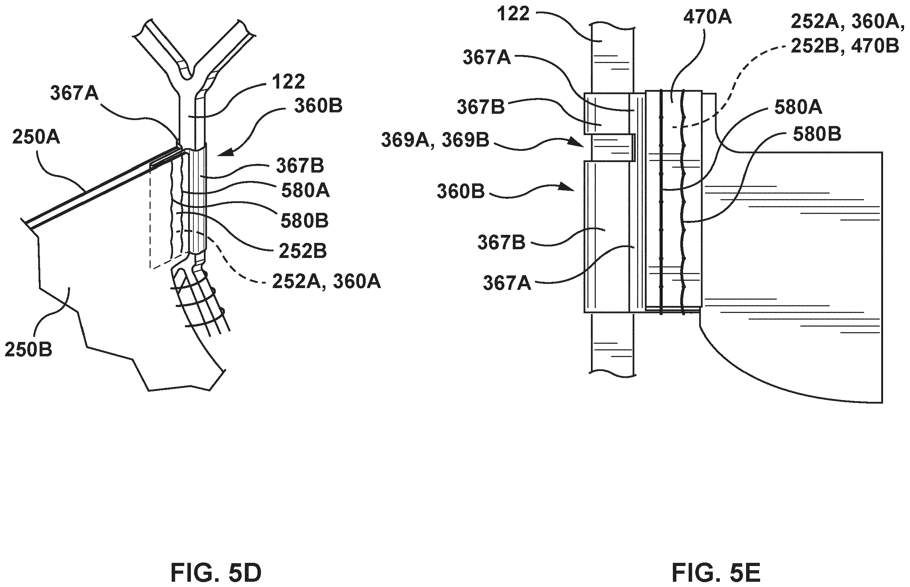

FIG. 5 is a block diagram of a method 500 of assembling a transcatheter heart valve prosthesis 100, which includes attaching the leaflets 250, commissure backings 360 and reinforcing members 470 of the prosthetic valve 140. The method as described is after the skirt 142 is attached to the frame 110 of the transcatheter heart valve prosthesis 100 and the base edge 256 of each leaflet 250 is attached to the skirt 142 and/or frame 110. However, this is not meant to be limiting regarding the order of operations. In the method shown and described herein, the prosthetic valve 140 of the transcatheter heart valve prosthesis 100 includes exactly three leaflets 250, exactly three commissure backings 360 and exactly three reinforcing members 470. However, this is not meant to be limiting, as one of skill in the art would understand that more or fewer components may be used, depending on the desired function and placement within the native heart. Further, as described in more detail below, the reinforcing members 470 are optional such that the method 500 need not include the steps directed to the tissue reinforcing members 470. Further, the method 500 will be described with respect to attaching the commissure formed by two adjacent leaflets 250. The steps of the method 500 would be repeated for the other commissures.

In a first step 501 of the method 500, the commissure backing 360 is folded along the fold line 361 into the folded configuration, as shown in FIG. 3B. As noted above, however, the commissure backing 360 is some embodiments need not be folded. In such embodiments, step 501 may be excluded.

In a second step 502 of the method 500, the commissure backing 360, in the folded configuration, is positioned between a first leaflet 250A and a second leaflet 250B adjacent to the first leaflet 250A, as illustrated by FIG. 5A. More particularly, the first portion 360A of the commissure backing 360 is positioned between and overlaps with the first lateral end 252A of the first leaflet 250A and the second lateral end 252B of the second leaflet 250B. Templates may be used to ensure proper alignment of the first portion 360A of the commissure backing 360, the first lateral end 252A of the first leaflet 250A, and the second lateral end 252B of the second leaflet 250B. The second portion 360B of the commissure backing 360 extends laterally from the first portion 360A of the commissure backing 360, the first lateral end 252A of the first leaflet 250A, and the second lateral end 252B of the second leaflet 250B, as can be seen in FIG. 5A. In other words, the second portion 360B of the commissure backing 360 does not overlap with the first lateral end 252A of the first leaflet 250A and the second lateral end 252B of the second leaflet 250B, as shown in FIG. 5A, but rather extends out laterally from the first portion 360A of the commissure backing 360. The first and second leaflets 250A, 250B extend out laterally from the first portion 360A of the commissure backing 360 in an opposite direction of the section portion 360B of the commissure backing 360, as can be seen in FIG. 5A. In the context of the frame 110 of the transcatheter heart valve prosthesis 100, the second portion 260B of the commissure backing 360 extends radially away from the central longitudinal axis L_{A}, and the first and second leaflets 250A, 250B extend radially toward the central longitudinal axis L_{A}, although they curve away from the central longitudinal axis to meet the corresponding adjacent leaflets at their corresponding commissures, as would be understood by those skilled in the art.

In a next step 503A of the method 500, the first portion 360A of the commissure backing 360 is attached or joined together to the first lateral end 252A of the first leaflet 250A and the second lateral end 252B of the second leaflet 250B. The first portion 360A of the commissure backing 360, the first lateral end 252A of the first leaflet 250A and the second lateral end 252B of the second leaflet 250B can be joined together, for example, using sutures. A single stitch line 580A or 580B or two parallel stitch lines 580A, 580B may be used to attach the first portion 360A of the commissure backing 360 to the first and second lateral ends 252A, 252B of the first and second leaflets 250A, 250B. The one or more stitch lines 580A, 580B run along an entire longitudinal length of the first portion 360A of the commissure backing 360, the first lateral end 252A of the first leaflet 250A and the second lateral end 252B of the second leaflet 250B. FIG. 5C shows this attachment but in an embodiment of the method including the reinforcing member 470, as described below. However, the step 503A may be identical to that shown in FIG. 5C except that the reinforcing member 470 is removed.

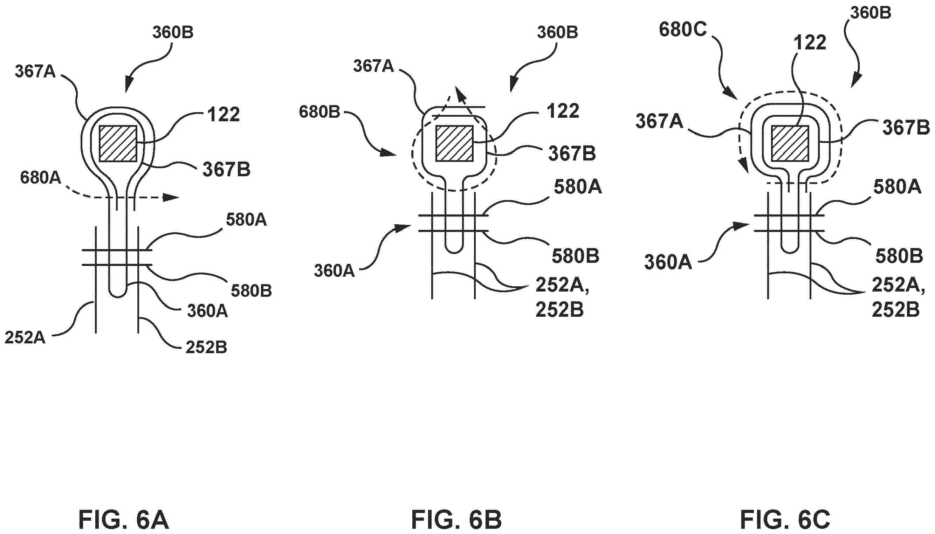

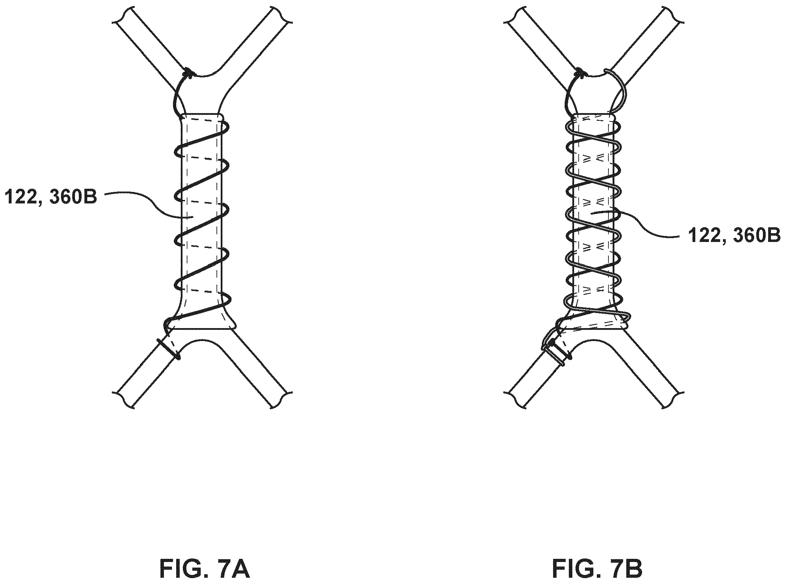

In a next step 504A of the method 500, the second portion 360B of the commissure backing 360 is attached to a commissure post 122 of the frame 110 of the transcatheter heart valve prosthesis 100. Because the commissure backing 360 is folded in half, the commissure backing 360 includes a first end 367A and a second end 367B. The first end 367A of the commissure backing 360 is referred to herein as the segment that extends from the fold line 361 to the first side edge 366 of the commissure backing 360, and the second end 367B of the commissure backing 360 is referred to herein as the segment that extends from the fold line 361 to the second side edge 368 of the commissure backing 360. The second end 367B of the commissure backing 360 aligns and overlaps with the first end 367A of the commissure backing 360. Accordingly, the first end 367A and the second end 367B of the second portion 360B of the commissure backing 360 are wrapped around a commissure post 122 of the frame 110, as shown in FIG. 5D. Then, the second portion 360B of the commissure backing 360 is attached to the commissure post 122 of the frame 110, for example, by using sutures. The second portion 360B of the commissure backing 360 is attached to the commissure post 122 of the frame 110 such that the first portion 360A of the commissure backing 360 and the first and second lateral ends 252A, 252B of the first and second leaflets 250A, 250B are disposed within the interior surface of the frame 110. Various methods of attaching the second portion 360B of the commissure backing 360 to a commissure post 122 of the frame 110 will be shown and described with respect to FIGS. 6A-6F and FIGS. 7A-7B.

The commissure backing 360 provides an increase in commissure material strength and reduces the additional bulk of a tissue-based approach by utilizing thinner fabrics. This allows for an increase in valve opening area and increases the durability of the commissures, thereby improving the effective orifice area (EOA) and durability of the valve.

Each of the steps shown and described herein may be repeated for the number of leaflets included within the prosthetic valve 140 of the transcatheter heart valve prosthesis 100. In the embodiment shown and described herein, the steps above are repeated to join the second leaflet 250B to a third leaflet 250C and to join the third leaflet 250C to the first leaflet 250A in an identical or similar manner. However, this is not meant to be limiting, as the method may be repeated more or fewer times to achieve the same goal.

As mentioned previously, the prosthetic valve 140 of the transcatheter heart valve prosthesis 100 may optionally include one or more reinforcing members 470, shown and described with respect to FIGS. 4A-4B. In such an embodiment, the method of assembling the transcatheter heart valve prosthesis 100 includes the same first and second steps 501, 502 of the method 500 described above. However, the following steps slightly differ from the method of assembling the transcatheter heart valve prosthesis 100 when the prosthetic valve 140 does not include the reinforcing members 470, which will be described in detail below.

In an embodiment where the prosthetic valve 140 of the transcatheter heart valve prosthesis 100 includes the reinforcing members 470, a next step 503B of the method 500, after steps 501 and 502 described above, includes positioning a reinforcing member 470 in the folded configuration over the first portion 360A of the commissure backing 360 and the first and second lateral ends 252A, 252B of the first and second leaflets 250A, 250B. More particularly, the reinforcing member 470 is placed over the first portion 360A of the commissure backing 360 and the first and second lateral ends 252A, 252B of the first and second leaflets 250A, 250B such that the first portion 470A of the reinforcing member 470 overlaps against the first lateral end 252A of the first leaflet 250A and the second portion 470B of the reinforcing member 470 overlaps against the second lateral end 252B of the second leaflet 250B, as shown in FIG. 5B. Templates may be used to ensure proper alignment of the reinforcing member 470, the first portion 360A of the commissure backing 360, the first lateral end 252A of the first leaflet 250A, and the second lateral end 252B of the second leaflet 250B. The reinforcing member 470 is folded at the fold line 471. The fold line 471 of the reinforcing member 470 is disposed at the upper edge 362 of the first portion 360A of the commissure backing 360, as can be seen in FIG. 5B. The upper and lower edges of the reinforcing members 470 align or overlap with the lower edge 364 of the first portion 360A of the commissure backing, as shown in FIG. 5B.

In a next step 504B of the method 500, the folded reinforcing member 470 is attached to the first portion 360A of the commissure backing 360, the first lateral end 252A of the first leaflet 250A and the second lateral end 252B of the second leaflet 250B, as shown in FIG. 5C. The reinforcing member 470, the first portion 360A of the commissure backing 360, and the first and second lateral ends 252A, 252B of the first and second leaflets 250A, 250B can be joined together, for example, using sutures 580A, 580B. A single stitch line 580A or 580B or two parallel stitch lines 580A, 580B may be used to attach the reinforcing member 470 to the first portion 360A of the commissure backing 360 and the first and second lateral ends 252A, 252B of the first and second leaflets 250A, 250B. Preferably, two parallel stitch lines 580A, 580B are used to join the reinforcing member 470 to the first portion 360A of the commissure backing 360 and the first and second lateral ends 252A, 252B of the first and second leaflets 250A, 250B. The one or more stitch lines 580A, 580B run along an entire longitudinal length of the first and second portions 470A, 470B of the reinforcing member 470, the first portion 360A of the commissure backing 360, and the first and second lateral ends 252A, 252B of the first and second leaflets 250A, 250B, as shown in FIG. 5C.

In a next step 505B of the method 500, the second portion 360B of the commissure backing 360 is attached to a commissure post 122 of the frame 110 of the transcatheter heart valve prosthesis 100. To do this, the first end 367A and the second end 367B of the second portion 360B of the commissure backing 360 are wrapped around a commissure post 122 of the frame 110, as shown in FIG. 5E. Then, the second portion 360B of the commissure backing 360 is attached to the commissure post 122 of the frame 110, for example, by using sutures. The second portion 360B of the commissure backing 360 is attached to the commissure post 122 of the frame 110 such that the first portion 360A of the commissure backing 360, the reinforcing member 470 and the first and second lateral ends 252A, 252B of the first and second leaflets 250A, 250B are disposed within the interior surface of the frame 110. Various methods of attaching the second portion 360B of the commissure backing 360 to a commissure post 122 of the frame 110 will be shown and described with respect to FIGS. 6A-6F and FIGS. 7A-7B.

The commissure backing 360 and the reinforcing member 470 provide an increase in commissure material strength and reduces the additional bulk of a tissue-based approach by utilizing thinner fabrics. This allows for an increase in valve opening area and increases the durability of the commissures, thereby improving the effective orifice area (EOA) and durability of the valve.

In some embodiments, the commissure posts 122 of the frame 110 may have one or more struts 116 that extend from a distal portion of each of the commissure posts 122, and may have a distal extension of the commissure post 122 that is distal of where the distal struts 116 intersect with the commissure posts 122. In such an embodiment, the one or more cutouts 369A, 369B of the commissure backing 360 are configured to allow the one or more struts 116 connected to the commissure post 122 to extend from the commissure post 122 without interrupting the attachment of the second portion 360B of the commissure backing 360. For example, the first and second ends 367A, 367B of the second portion 360B of the commissure backing 360 that are disposed distal of the cutouts 369A, 369B, i.e., nearest the outflow end 104 of the transcatheter heart valve prosthesis 100, would wrap around the commissure post 122 distal of where the one or more distal struts 116 intersect with the commissure post 122. Similarly, the first and second ends 367A, 367B of the second portion 360B of the commissure backing 360 that are disposed proximal of the cutouts 369A, 369B, i.e., nearest the inflow end 102 of the transcatheter heart valve prosthesis 100, would wrap around the commissure post 122 proximal of where the one or more struts 116 intersect with the commissure posts 122. The cutouts 369A, 369B shown and described herein are substantially rectangular-shaped, however this is not meant to be limiting, as any cutout size or shape known to those skilled in the art may be used such that the second portion 360B of the commissure backing 360 may wrap around and connect to the commissure post 122 of the frame 110 without interruption.

Each of the steps shown and described herein may be repeated for the number of leaflets included within the prosthetic valve 140 of the transcatheter heart valve prosthesis 100. For example, in the embodiment shown and described herein, the steps above are repeated to attach or join the second leaflet 250B to the third leaflet 250C, and to attach or join the third leaflet 250C to the first leaflet 250A in an identical or similar manner. However, this is not meant to be limiting, as the method may be repeated more or fewer times to achieve the same goal.

FIGS. 6A-6F and 7A-7B show various ways the second portion 360B of the commissure backing 360 can be attached or joined to a commissure post 122 of the frame 110 of the transcatheter heart valve prosthesis 100, according to embodiments hereof. FIGS. 6A-6F show commissure attachments as viewed from the outflow end 104 of the transcatheter heart valve prosthesis 100.

For example, FIG. 6A shows the first and second ends 367A, 367B of the second portion 360B of the commissure backing 360 wrapped around both sides of the commissure post 122 until the first and second ends 367A, 367B wrap fully around the commissure post 122 and extend back towards the first portion 360A of the commissure backing 360. A running stitch line 680A extend through the base and ends of the first and second ends 367A, 367B of the second portion 360B of the commissure backing 360 at a location between the commissure post 122 and the first portion 360A of the commissure backing 360 that is attached to the lateral ends 252A, 252B of the first and second leaflets 250A, 2520B, as shown in FIG. 6A. In other words, the stitch line 680A is disposed between the lateral ends 252A, 252B and the commissure post 122. This suture pattern may be repeated along an entire longitudinal length of the commissure post 122 and the second portion 360B of the commissure backing 360.

FIG. 6B illustrates another example, wherein the first and second ends 367A, 367B of the second portion 360B of the commissure backing 360 are wrapped around both sides of the commissure post 122 such that the ends 367A, 367B overlap at an outer or exterior surface of the commissure post 122, i.e., the surface of the commissure post 122 that faces away from the interior surface of the frame 110. In this example, a suture 680B is run through the first and second ends 367A, 367B where they overlap at the exterior surface of the commissure post 122, extending the suture through the first end 367A of the commissure backing 360, wrapping the suture around and through the first and second ends 367A, 367B of the second portion 360B of the commissure backing 360 at a location between the commissure post 122 and the first portion 360A of the commissure backing 360, continuing to wrap the suture around the commissure post 122, and extending the suture 680B back through the first and second ends 367A, 367B of the commissure backing 360 at the exterior surface of the commissure post 122. This suture pattern may be repeated along an entire longitudinal length of the commissure post 122 and the second portion 360B of the commissure backing 360.

FIG. 6C illustrates an alternate example, wherein the first and second ends 367A, 367B of the second portion 360B of the commissure backing 360 wrap around both sides of the commissure post 122 until the first and second ends 367A, 367B wrap fully around the commissure post 122 and extend back towards the first portion 360A of the commissure backing 360. In this example, a suture 680C is run through the base and ends of both the first and second ends 367A, 367B of the second portion 360B of the commissure backing 360 at a location between the commissure post and the first portion 360A of the commissure backing, as shown in FIG. 6C. Then, the suture 680C is wrapped around the exterior of the commissure post 122 and the first and second ends 367A, 367B of the commissure backing 360. The suture 680C is then run up or down the commissure post 122 to another location where the suture 608C is extended through the ends 367A, 367B between the commissure post 122 and the first portion 360A, and wrapped around the exterior of the commissure post 122 and the first and second ends 367A, 367B, as described. This suture pattern may be repeated along an entire longitudinal length of the commissure post 122 and the second portion 360B of the commissure backing 360.

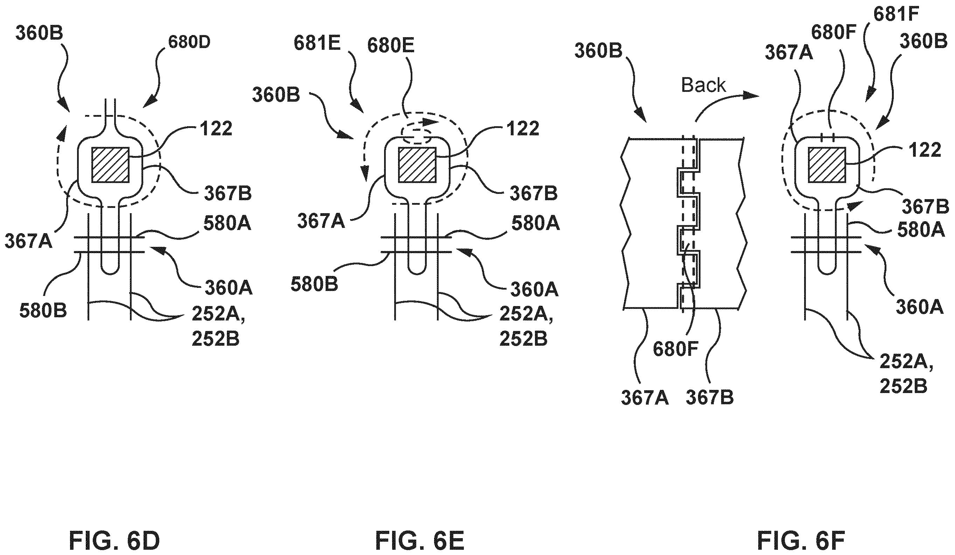

FIG. 6D shows an alternate example, wherein the first and second ends 367A, 367B of the second portion 360B of the commissure backing 360 wrap around opposite sides of the commissure post 122 until they meet at the exterior surface of the commissure post 122. In this example, a suture 680B is run through the first and second ends 367A, 367B of the commissure backing 360 at the exterior surface of the commissure post 122, the suture 680B is wrapped around the commissure post 122 and extends through the first and second ends 367A, 367B of the second portion 360B of the commissure backing 360 at a location between the commissure post 122 and the first portion 360A of the commissure backing 360, as shown in FIG. 6D. Then, the suture 680B continues to be wrapped around the rest of the commissure post 122. This suture pattern may be repeated along an entire longitudinal length of the commissure post 122 and the second portion 360B of the commissure backing 360.

FIG. 6E shows an alternate example, wherein the first and second ends 367A, 367B of the second portion 360B of the commissure backing 360 wrap around opposite sides of the commissure post 122 until they meet at the exterior surface of the commissure post 122. In this example, a first suture 680E attaches the first end 367A of the commissure backing 360 to the second end 367B of the commissure backing 360 at the exterior surface of the commissure post 122, as shown in FIG. 6E. In other words, the first end 367A and the second end 367B of the commissure backing 360 are stitched to each other along their longitudinal length at the exterior surface of the commissure post 122. A second suture 681E extends through the first and second ends 367A, 367B of the commissure backing 360 at a location between the commissure post 122 and the first portion 360A of the commissure backing 360. The second suture 681E is then wrapped around the entire commissure post 122 and the first and second ends 367A, 367B of the second portion 360B of the commissure backing 360. This suture pattern may repeated along an entire longitudinal length of the commissure post 122 and the second portion 360B of the commissure backing 360.

FIG. 6F shows another example, wherein the first and second ends 367A, 367B of the second portion 360B of the commissure backing 360 include alternating protrusions and depressions such that the first and second ends 367A, 367B align in a zipper-like configuration. The first and second ends 367A, 367B of the second portion 360B of the commissure backing 360 wrap around both sides of the commissure post 122 until they meet at the exterior surface of the commissure post 122. In this example, in a first operation, the first and second ends 367A, 367B are aligned in the zipper-like configuration and a first suture 680F is stitched longitudinally to secure the first and second ends 367A, 367B to one another. The suture 680F can coupled the first and second ends 367A, 367B together along a single stitch line or two parallel stitch lines, or other stitching patterns. In other words, the first end 367A and the second end 367B of the commissure backing 360 are stitched to each other along their entire longitudinal length at the exterior surface of the commissure post 122. In a second operation, a second suture 681F extends through the first and second ends 367A, 367B of the commissure backing 360 at a location between the commissure post 122 and the first portion 360A of the commissure backing 360. The second suture 681F is then wrapped around the entire commissure post 122 and the first and second ends 367A, 367B of the commissure backing 360. This suture pattern is repeating along the longitudinal length of the commissure post 122 and the second portion 360B of the commissure backing 360, such as along the entire longitudinal length.

Each of the steps shown and described herein may be repeated depending on the number of commissure backings 360 that are to be attached to the commissure posts 122 of the transcatheter heart valve prosthesis 100. For example, in the embodiment shown and described herein, the steps above are repeated a total of three times such that exactly three commissure backings are coupled to exactly three commissure posts in an identical or similar manner. However, this is not meant to be limiting, as the method may be repeated more or fewer times to achieve the same goal.

FIGS. 7A-7B show a side view of a commissure post 122 after the second portion 360B of the commissure backing 360 has been attached to the commissure post 122. FIG. 7A shows an example of a single loop stitch that wraps around the commissure post 122 and the second portion 360B of the commissure backing 360 and traverses the entire longitudinal length of the commissure post 122. FIG. 7B shows an example of a "shoelace" stitch pattern that utilizes two sutures that repeatedly cross over one another, or "crisscross", around the commissure post 122 and the second portion 360B of the commissure backing 360 along the entire longitudinal length of the commissure post 122.

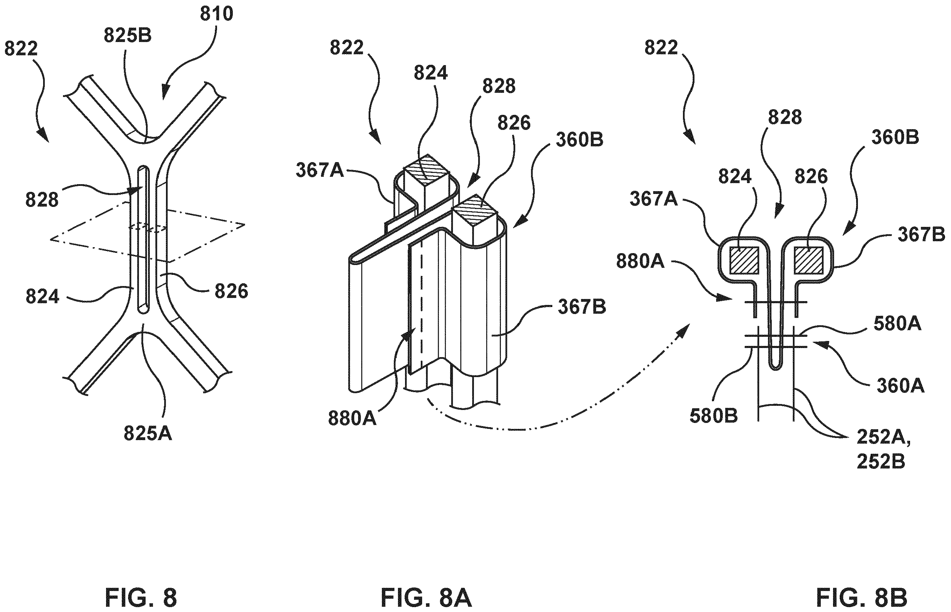

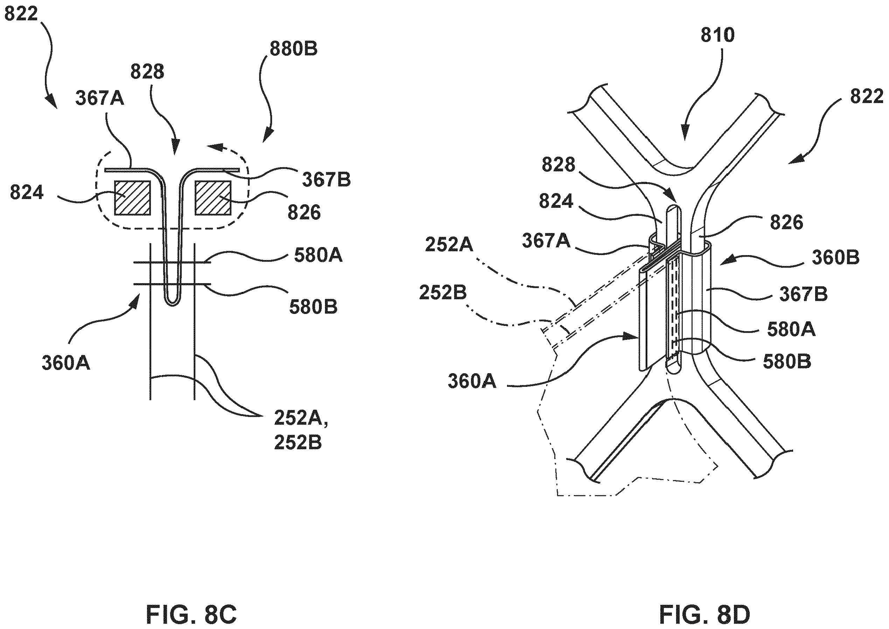

In some embodiments, the frame 110 of the transcatheter heart valve prosthesis 100 may include one or more slot commissure posts 822, as shown in FIG. 8. The slot commissure post 822 includes a first axial strut 824 and a second axial strut 826 that runs parallel and is adjacent to the first axial strut 824. Each of the first and second axial struts 824, 826 include an inflow end, *i.e.,* an end that is nearest the inflow end 102 of the prosthesis 100, and an outflow end, *i.e.,* an end that is nearest the outflow end 104 of the prosthesis 100. The inflow ends of the first and second axial struts 824, 826 meet and connect at a first node 825A, and the outflow ends of the first and second axial struts 824, 826 meet and connect at a second node 825B, as shown in FIG. 8. An opening or slot 828 of the slot commissure post 822 is defined between the first and second axial struts 824, 826 and the first and second nodes 825A, 825B. In such an embodiment, the first and second ends 367A, 367B of the second portion 360B of the commissure backing 360 are secured to the first and second axial struts 824, 826 of the slot commissure post 822. FIGS. 8A-8D show various examples of how the second portion 360B of the commissure backing 360 can be attached to a slot commissure post 822 of a frame.

In a first example shown in FIGS. 8A-8B, the first portion 360A of the commissure backing 360 is oriented and coupled between the first and second lateral ends 252A, 252B of the first and second leaflets 250A, 250B, as shown and described above with reference to FIGS. 5A-5E. Then, the first and second ends 367A, 367B of the second portion 360B of the commissure backing 360 are extended radially outwardly through the opening 828 of the slot commissure post 822, as shown in FIG. 8A. More particularly, the first and second ends 367A, 367B of the second portion 360B of the commissure backing 360 are extended through the opening 828 in a direction from the interior of the frame 810 to the exterior of the frame 810 such that the first portion 360A of the commissure backing 360 and the first and second lateral ends 252A, 252B of the first and second leaflets 250A, 250B are disposed within the interior surface of the frame 810. Next, the first end 367A of the commissure backing 360 extends circumferentially away from the opening 828 and around the first axial strut 824 such that the first end 367A is wrapped around the first axial strut 824 of the slot commissure post 822 until the first end 367A extends back towards the first portion 360A of the commissure backing 360, as shown in FIGS. 8A-8B. Similarly, the second end 367B of the commissure backing 360 extends through the opening 828, circumferentially away from the opening 828 and around the second axial strut 826 such that the second end 367B is wrapped around the second axial strut 826 of the slot commissure post 822 until the second end 367B extends back towards the first portion 360A of the commissure backing 360. The first and second ends 367A, 367B can then be secured to each other and the slot commissure bar 822 by extending a suture 880A through two layers of the first end 367A and two layers of the second end 367B between the slot commissure post 822 and the first and second lateral ends 252A, 252B of the first and second leaflets 250A, 250B. The suture 880A forms a stitch line longitudinally along the length of the first and second end 367A, 367B, as shown in FIG. 8A. Although shown as a single running stitch, other stitch patterns, including but not limited to a double running stitch, may be utilized.

FIG. 8C illustrates another example, wherein the first and second ends 367A, 367B of the second portion 360B of the commissure backing 360 are extended radially outwardly through the opening 828 of the slot commissure post 822. The first end 367A then extends circumferentially away from the opening 828 in a first direction along the exterior surface of the first axial strut 824. The first end 367A does not extend all the way around the first axial strut 824 as in FIGS. 8A-8B. Similarly, after extending radially outwardly through the opening 828, the second end 367B extends circumferentially in a second direction, opposite the first direction, away from the opening 828 and along the exterior surface of the second axial strut 826. As with the first end 367A, the second end 367B does not extend all the way around the second axial strut 826. The first and second ends 367A, 367B are then coupled to each other and the slot commissure post 822 by extending a suture 880B through the first end 367A adjacent the free end thereof and adjacent the exterior surface of the first axial strut 824. The suture 880B is wrapped around the first axial strut 824, through the first and second ends 367A, 367B between the interior surface of the slot commissure post and the first and second lateral ends 252A, 252B, around the second axial strut 826, and through the second end 367B adjacent the free end thereof and adjacent the exterior surface of the second axial strut 826, as shown in FIG. 8C. This suture pattern is repeated along an entire longitudinal length of the slot commissure post 822 and the second portion 360B of the commissure backing 360.

FIG. 8D shows the slot commissure post 822 of the frame 110 after the second portion 360B of the commissure backing 360 is attached to the slot commissure post 822. As can be seen, the second portion 360B of the commissure backing 360 is attached to the slot commissure post 822 of the frame 810 such that the first portion 360A of the commissure backing 360 and the first and second lateral ends 252A, 252B of the first and second leaflets 250A, 250B are disposed within the interior surface of the frame 810.

Each of the steps shown and described herein may be repeated depending on the number of commissures that are to be attached to commissure posts of the transcatheter heart valve prosthesis 100. For example, in the embodiment shown and described herein, the steps above are repeated a total of three times such that exactly three commissure backings are coupled to exactly three slot commissure posts in an identical or similar manner. However, this is not meant to be limiting, as the method may be repeated more or fewer times to achieve the same goal.

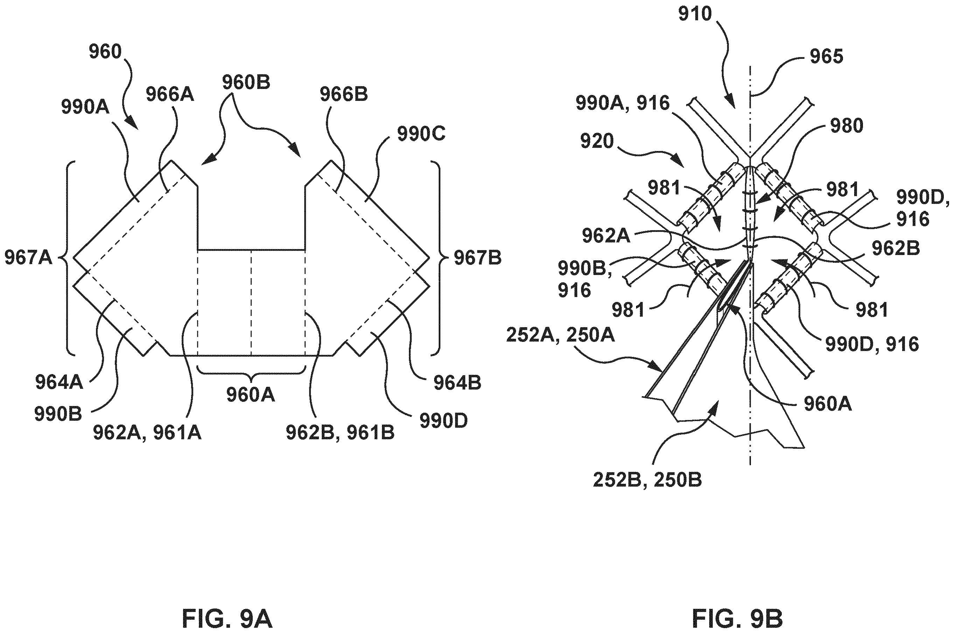

In another embodiment, the frame 910 of the transcatheter heart valve prosthesis 900 does include commissure posts 122 or slot commissure posts 822 as described above. Instead, the commissures of the prosthetic valve 940 are attached to the frame 910 of the prosthesis 900 at open cells 920 of the frame 910. FIG. 9A shows an embodiment of a commissure backing 960 in an as-cut state that is configured to be attached to an open cell 920 of the frame 910. The commissure backing 960 includes a first portion 960A and a second portion 960B. The first portion 960A of the commissure backing 960 is substantially rectangular-shaped and configured to fold in half and attach to the first and second lateral ends 252A, 252B of the first and second leaflets 250A, 250B, similar to the commissure backing 360 shown and described in the previous embodiments.

The second portion 960B of the commissure backing 960 further includes a first end 967A configured to attach to two adjacent struts 916 of the four total struts 916 that define the open cell 920 of the frame 910, and a second end 967B configured to attach to the remaining two adjacent struts 916 of the four total 916 that define the open cell 920 of the frame 910, as shown in FIG. 9B. The first end 967A of the commissure backing 960 may be substantially triangle-shaped with three edges. A first edge 962A is coupled to or integrally formed with a first side edge 961A of the first portion 960A of the commissure backing 960. The second and third edges 964A, 966A of the first end 967A include first and second tabs 990A, 990B that extend outward from the respective second and third straight edges 964A, 966A of the first end 967A. The first and second tabs 990A, 990B may substantially rectangular-shaped and are configured to wrap around two adjacent struts 916 of the four total struts 916 of the open cell 920, as shown in FIG. 9B.

Similarly, the second end 967B of the commissure backing 960 is substantially triangle-shaped with three edges. A first edge 962B is coupled to or integrally formed with a second side edge 961B of the first portion 960A of the commissure backing 960. Second and third edges 964B, 966B of the second end 967B include third and fourth tabs 990C, 990D that extend outward from the second and third straight edges 964B, 966B of the second end 967B. The third and fourth tabs 990C, 990D may be substantially rectangular-shaped and are configured to wrap around the other two adjacent struts 916 of the four total struts 916 of the open cell 920, as shown in FIG. 9B.

To attach the commissure backing 960 to an open cell 920 of the frame 910, a step includes folding the first portion 960A of the commissure backing 960 in half such that the first and second side edges 961A, 961B of the first portion 960A meet. As in the embodiments described above, the first portion 960A is disposed between and is attached to the first and second end tabs 2502A, 252B of the first and second leaflets 250A, 250B. In another step, the first edges 962A, 962B of the first and second ends 967A, 967B are coupled to each other via a suture 980. It is noted that in this embodiment, a portion of the first edges 962A, 962B of the first and second ends 967A, 967B are shared with the first and second of side edges 931A, 931B of the first portion 960A. The first edges 962A, 962B of the first and second ends 967A, 967B (together with the first and second side edges 961A, 961B of the first portion 960A) are located at a center of the open cell 920. More specifically, the first edges 962A, 962B of the first and second ends 967A, 967B extend longitudinally within the open cell 920 along a central longitudinal axis 965 located centrally within the cell 920, as shown in FIG. 9B. In another step, the first end 967A extends from the central longitudinal axis 965 circumferentially in a first direction within the cell 920 and the second end 967B extends from the central longitudinal axis 965 circumferentially in a second direction within the cell 920 opposite the first direction. Due to the shape of the first and second ends 967A, 967B matching the shape of the cell 920, the each of the four tabs 990A-990D is disposed adjacent a respective one of the struts 916 defining the cell 920. In another step, each tab 990A-990D is wrapped around its respective strut 916 and is attached to the strut using suture(s) 981, as shown in FIG. 9B. Attaching the four tabs 990A-990D to the four struts 916 of the open cell 920 securely attaches the second portion 960B of the commissure backing 960 to the open cell 920 of the frame 910.

Each of the steps shown and described herein may be repeated depending on the number of commissures that are to be attached to open cells of the transcatheter heart valve prosthesis. For example, in the embodiment shown and described herein, the steps above are repeated a total of three times such that exactly three commissure backings are coupled to exactly three open cells of the frame in an identical or similar manner. However, this is not meant to be limiting, as the method may be repeated more or fewer times to achieve the same goal. Further, although described in a particular order, the steps need not be performed in the order described.

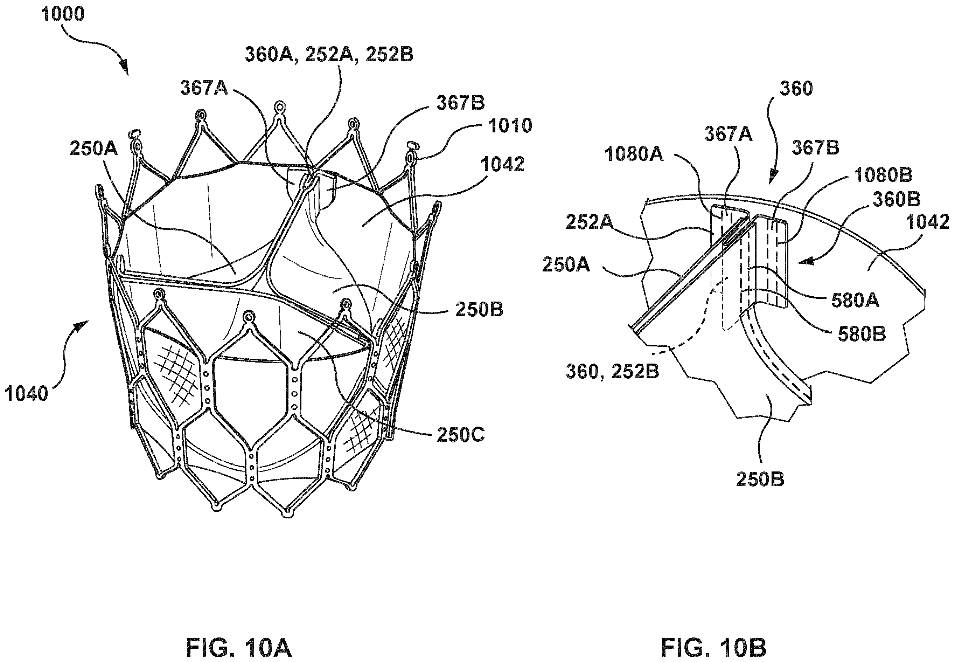

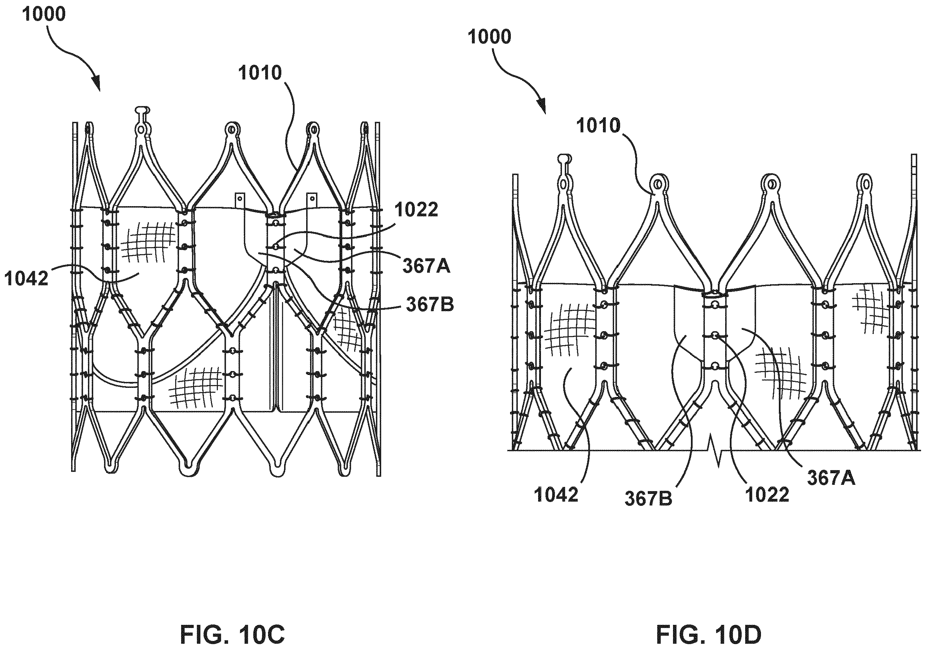

In another embodiment, as shown and described herein with respect to FIGS. 10A-10D, the second portion 360B of the commissure backing 360 shown and described above with respect to FIGS. 3A-3B may be attached or joined to the skirt 1042 of the transcatheter heart valve prosthesis 1000. As can be seen in the perspective view of the transcatheter heart valve prosthesis 1000 in FIG. 10A, the skirt 1042 in this embodiment lines a majority of the interior surface of the frame 1010. The base edge 256 of each leaflet 250 of the prosthetic valve 1040 is attached to the skirt 1042 via sutures. The first portion 360A of the commissure backing 360 is disposed between and is attached to the first and second lateral ends 252A, 252B of the first and second leaflets 250A, 250B. The first portion 360A of the commissure backing 360 is attached to the first and second lateral ends 252A, 252B of the first and second leaflets 250A, 250B via two parallel stitch lines 580A, 580B that may extend an entire longitudinal length of the first portion 360A of the commissure backing 360, as described above.

The first and second ends 367A, 367B of the second portion 360B of the commissure backing 360 are shown extending circumferentially in opposite directions along an interior surface of the skirt 1042. The first end 367A of the commissure backing 360 is then attached to the interior surface of the skirt 1042 via two parallel stitch lines 1080A that may extend an entire longitudinal length of the first end 367A of the commissure backing 360. Similarly, the second end 367B of the commissure backing 360 is attached to the interior surface of the skirt 1042 via two parallel stitch lines 1080B that may extend an entire longitudinal length of the second end 367B of the commissure backing 360, as shown in FIG. 10B. Although described as two pairs of parallel stitch lines 1080A, 1080B, this is not meant to be limiting, and other stitch patterns may be used.

FIGS. 10C-10D show a side view of the transcatheter heart valve prosthesis 1000. As can be seen, additional sutures may be used to attach the first and second ends 367A, 367B of the commissure backing 360 and the skirt 1042 to one or more commissure posts 1022 of the frame 1010.

Each of the steps shown and described herein may be repeated depending on the number of commissures that are to be attached to commissure posts of the transcatheter heart valve prosthesis. For example, in the embodiment shown and described herein, the steps above are repeated a total of three times such that exactly three commissure backings are coupled to exactly three commissure posts in an identical or similar manner. However, this is not meant to be limiting, as the method may be repeated more or fewer times to achieve the same goal.

Further, many of the embodiments described above do not show the reinforcing member. However, this is not meant to be limiting. The reinforcing member was not shown in these embodiments for sake of clarity of the drawings. Each of the embodiments described can include a reinforcing member as described with respect to FIGS. 4A-4B and FIGS. 5B, 5C, and 5E.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

Further disclosed herein is the subject-matter of the following clauses:
1. A transcatheter heart valve prosthesis comprising:
   a frame;
   a prosthetic valve having a plurality of leaflets, each leaflet including lateral ends; and
   a commissure backing having:
      a first portion disposed between a first lateral end of a first leaflet of the plurality of leaflets and a second lateral end of a second leaflet of the plurality of leaflets, wherein the first lateral end, the second lateral end, and the first portion of the commissure backing are joined together, and
      a second portion that extends radially outwardly from the first and second lateral ends, wherein the second portion of the commissure backing is coupled to the frame.
2. The transcatheter heart valve prosthesis of clause 1, wherein the transcatheter heart valve prosthesis further includes a reinforcing member, wherein the reinforcing member is joined to the first lateral end, second lateral end, and the first portion of the commissure backing.
3. The transcatheter heart valve prosthesis of clause 2, wherein the reinforcing member comprises tissue, polytetrafluoroethylene (PTFE), hydrogels, and/or polymers.
4. The transcatheter heart valve prosthesis of clause 2 or of any of clauses 2-3, wherein the reinforcing member includes a fold.
5. The transcatheter heart valve prosthesis of clause 4, wherein the reinforcing member includes a first portion disposed on an outer surface of the first lateral end of the first leaflet and a second portion disposed on an outer surface of the second lateral end of the second leaflet, wherein the fold is disposed between the first and second portions of the leaflet, and wherein the reinforcing member is joined to the first lateral end, the second lateral end, and the first portion of the commissure backing via sutures extending from the first portion of the reinforcing member to the second portion of the reinforcing member through the first lateral end, the second lateral end, and the first portion of the commissure backing.
6. The transcatheter heart valve prosthesis of clause 1 or of any of the preceding clauses, wherein the commissure backing is folded such that the first portion of the commissure backing comprises two layers disposed between first lateral end of the first leaflet and the second lateral end of the second leaflet.
7. The transcatheter heart valve prosthesis of clause 1 or of any of the preceding clauses, wherein the commissure backing includes at least one cutout configured to allow a strut of the frame to extend therethrough.
8. The transcatheter heart valve prosthesis of clause 1 or of any of the preceding clauses, wherein the frame includes a slot commissure post, wherein the slot commissure post includes a first axial strut, a second axial strut, and an opening disposed between the first and second axial struts.
9. The transcatheter heart valve prosthesis of clause 8, wherein the commissure backing is folded such that the first portion of the commissure backing comprises two layers disposed between first lateral end of the first leaflet and the second lateral end of the second leaflet, and the second portion of the commissure backing includes a first end extending from the fold and a second end extending from the fold.
10. The transcatheter heart valve prosthesis of clause 9, wherein the second portion of the commissure backing extends radially outwardly through the opening of the slot commissure post such that the first portion of the commissure backing, the first lateral end of the first leaflet, and the second lateral end of the second leaflet are disposed within an interior of the frame.
11. The transcatheter heart valve prosthesis of clause 10, wherein the second portion of the commissure backing is wrapped around the first axial strut and second axial strut of the slot commissure post.
12. The transcatheter heart valve prosthesis of clause 1 or of any of the preceding clauses, wherein the commissure backing comprises polyethylene terephthalate (PET), polytetrafluoroethylene (PTFE), and/or polyester.
13. A method of assembling a transcatheter heart valve prosthesis comprising:
   positioning a first portion of a commissure backing between a first lateral end of a first leaflet and a second lateral end of a second leaflet, the second leaflet being adjacent to the first leaflet, wherein a second portion of the commissure backing extends radially outwardly from the first portion;
   attaching the first portion of the commissure backing to the first lateral end of the first leaflet and the second lateral end of the second leaflet; and
   attaching the second portion of the commissure backing to a commissure post of a frame.
14. The method of clause 13, further comprising attaching a reinforcing member to the first lateral end of the first leaflet, the second lateral end of the second leaflet, and the first portion of the commissure backing.
15. The method of clause 14, wherein attaching the reinforcing member comprises positioning a first portion of the reinforcing member on an outer surface of the first lateral end of the first leaflet, positioning a second portion of the reinforcing member on an outer surface of the second lateral end of the second leaflet, with a fold disposed between the first and second portions of the reinforcing member, and joining the reinforcing member to the first lateral end, the second lateral end, and the first portion of the commissure backing via sutures extending from the first portion of the reinforcing member to the second portion of the reinforcing member through the first lateral end, the second lateral end, and the first portion of the commissure backing.
16. The method of clause 13 or of any of clauses 13-15, further comprising folding the commissure backing to form the first portion of the commissure backing and the second portion of the commissure backing.
17. The method of clause 13 or of any of clauses 13-16, further comprising aligning a cutout of the commissure backing with a strut of the frame such that the strut extends through the cutout.
18. The method of clause 13 or of any of clauses 13-17, wherein attaching the second portion of the commissure backing to the commissure post comprises attaching the second portion of the commissure backing to a slot commissure post that includes a first axial strut, a second axial strut, and an opening disposed between the first and second axial struts.
19. The method of clause 18, wherein attaching the second portion of the commissure backing to the slot commissure post comprises extending the second portion of the commissure backing extends radially outwardly through the opening of the slot commissure post such that the first portion of the commissure backing, the first lateral end of the first leaflet, and the second lateral end of the second leaflet are disposed within an interior of the frame.
20. The method of clause 19, further comprising wrapping the second portion of the commissure backing at least partially around the first axial strut and second axial strut of the slot commissure post, and attached the second portion of the commissure backing to the slot commissure post.

## Claims

1. A transcatheter heart valve prosthesis comprising:
a frame;
a prosthetic valve having a plurality of leaflets, each leaflet including lateral ends; and
a commissure backing having:
a first portion disposed between a first lateral end of a first leaflet of the plurality of leaflets and a second lateral end of a second leaflet of the plurality of leaflets, wherein the first lateral end, the second lateral end, and the first portion of the commissure backing are joined together, and
a second portion that extends radially outwardly from the first and second lateral ends, wherein the second portion of the commissure backing is coupled to the frame.

2. The transcatheter heart valve prosthesis of claim 1, wherein the transcatheter heart valve prosthesis further includes a reinforcing member, wherein the reinforcing member is joined to the first lateral end, second lateral end, and the first portion of the commissure backing.

3. The transcatheter heart valve prosthesis of claim 2, wherein the reinforcing member comprises tissue, polytetrafluoroethylene (PTFE), hydrogels, and/or polymers.

4. The transcatheter heart valve prosthesis of any of claims 2-3, wherein the reinforcing member includes a fold.

5. The transcatheter heart valve prosthesis of claim 4, wherein the reinforcing member includes a first portion disposed on an outer surface of the first lateral end of the first leaflet and a second portion disposed on an outer surface of the second lateral end of the second leaflet, wherein the fold is disposed between the first and second portions of the leaflet, and wherein the reinforcing member is joined to the first lateral end, the second lateral end, and the first portion of the commissure backing via sutures extending from the first portion of the reinforcing member to the second portion of the reinforcing member through the first lateral end, the second lateral end, and the first portion of the commissure backing.

6. The transcatheter heart valve prosthesis of any of the preceding claims, wherein the commissure backing is folded such that the first portion of the commissure backing comprises two layers disposed between first lateral end of the first leaflet and the second lateral end of the second leaflet, and/or wherein the commissure backing includes at least one cutout configured to allow a strut of the frame to extend therethrough.

7. The transcatheter heart valve prosthesis of any of the preceding claims, wherein the frame includes a slot commissure post, wherein the slot commissure post includes a first axial strut, a second axial strut, and an opening disposed between the first and second axial struts.

8. The transcatheter heart valve prosthesis of claim 7, wherein the commissure backing is folded such that the first portion of the commissure backing comprises two layers disposed between first lateral end of the first leaflet and the second lateral end of the second leaflet, and the second portion of the commissure backing includes a first end extending from the fold and a second end extending from the fold.

9. The transcatheter heart valve prosthesis of claim 8, wherein the second portion of the commissure backing extends radially outwardly through the opening of the slot commissure post such that the first portion of the commissure backing, the first lateral end of the first leaflet, and the second lateral end of the second leaflet are disposed within an interior of the frame, wherein optionally the second portion of the commissure backing is wrapped around the first axial strut and second axial strut of the slot commissure post.

10. The transcatheter heart valve prosthesis of any of the preceding claims, wherein the commissure backing comprises polyethylene terephthalate (PET), polytetrafluoroethylene (PTFE), and/or polyester.

11. A method of assembling a transcatheter heart valve prosthesis comprising:
positioning a first portion of a commissure backing between a first lateral end of a first leaflet and a second lateral end of a second leaflet, the second leaflet being adjacent to the first leaflet, wherein a second portion of the commissure backing extends radially outwardly from the first portion;
attaching the first portion of the commissure backing to the first lateral end of the first leaflet and the second lateral end of the second leaflet; and
attaching the second portion of the commissure backing to a commissure post of a frame.

12. The method of claim 11, further comprising attaching a reinforcing member to the first lateral end of the first leaflet, the second lateral end of the second leaflet, and the first portion of the commissure backing, wherein optionally attaching the reinforcing member comprises positioning a first portion of the reinforcing member on an outer surface of the first lateral end of the first leaflet, positioning a second portion of the reinforcing member on an outer surface of the second lateral end of the second leaflet, with a fold disposed between the first and second portions of the reinforcing member, and joining the reinforcing member to the first lateral end, the second lateral end, and the first portion of the commissure backing via sutures extending from the first portion of the reinforcing member to the second portion of the reinforcing member through the first lateral end, the second lateral end, and the first portion of the commissure backing.

13. The method of any of claims 11-12, further comprising folding the commissure backing to form the first portion of the commissure backing and the second portion of the commissure backing, and/or further comprising aligning a cutout of the commissure backing with a strut of the frame such that the strut extends through the cutout.

14. The method of any of claims 11-13, wherein attaching the second portion of the commissure backing to the commissure post comprises attaching the second portion of the commissure backing to a slot commissure post that includes a first axial strut, a second axial strut, and an opening disposed between the first and second axial struts.

15. The method of claim 14, wherein attaching the second portion of the commissure backing to the slot commissure post comprises extending the second portion of the commissure backing extends radially outwardly through the opening of the slot commissure post such that the first portion of the commissure backing, the first lateral end of the first leaflet, and the second lateral end of the second leaflet are disposed within an interior of the frame, and optionally further comprising wrapping the second portion of the commissure backing at least partially around the first axial strut and second axial strut of the slot commissure post, and attached the second portion of the commissure backing to the slot commissure post.
